(19) Europäisches Patentamt
European Patent Office
Office européen des brevets

(11) EP 0 387 178 B2

(12) NEW EUROPEAN PATENT SPECIFICATION

(45) Date of publication and mention of the opposition decision:
**18.04.2001 Bulletin 2001/16**

(45) Mention of the grant of the patent:
**02.01.1997 Bulletin 1997/01**

(21) Application number: **90500023.8**

(22) Date of filing: **28.02.1990**

(51) Int Cl.[7]: **C12P 17/18**, C07D 498/04

# (54) Process for the preparation of clavulinic acid and pharmaceutically acceptable salts from fermentation broths of Streptomyces sp.

Verfahren zur Gewinnung von Klavulinsäure und deren pharmazeutisch annehmbare Salze aus Fermentationskulturen von Streptomyces sp.

Procédé de préparation de l'acide clavulinique et de ses sels pharmaceutiquement acceptables à partir de bouillons de fermentation de Streptomyces sp.

(84) Designated Contracting States:
**AT BE CH DE DK FR GB GR IT LI LU NL SE**

(30) Priority: **01.03.1989 ES 8900748**

(43) Date of publication of application:
**12.09.1990 Bulletin 1990/37**

(73) Proprietor: **SMITHKLINE BEECHAM PLC**
**Brentford, Middlesex TW8 9EP (GB)**

(72) Inventors:
- **De Urries Senante, Pilar J.**
  **E-28033 Madrid (ES)**
- **Garcia Alvarez, Concepción**
  **E-28002 Madrid (ES)**
- **Floriano Martin, Pablo**
  **E-28002 Madrid (ES)**

(74) Representative: **White, Susan Mary et al**
**SmithKline Beecham plc**
**Corporate Intellectual Property,**
**Two New Horizons Court**
**Brentford, Middlesex TW8 9EP (GB)**

(56) References cited:
**EP-A- 0 026 044** **EP-A- 0 182 522**
**ES-A- 2 010 143** **GB-A- 1 543 563**
**US-A- 4 140 764**

- **CRC Handbook of Chemistry and Physics, 65th Edition, 1984-1985 CRC Press, Inc., pages D-165 and D-166**
- **The Merck Index, tenth edition, 1983, Merck & Co., Inc page 863**
- **European Patents Handbook, second edition, Rel 21, 1965, pages 55/195 and 56/196**



Printed by Jouve, 75001 PARIS (FR)

## Description

### TECHNICAL FIELD OF THE INVENTION

Clavulanic acid of formula $C_8H_9NO_5$ whose systematic name is the one indicated in the title has broad spectrum antibiotic properties and its special interest lies in its inhibitory activity of β-lactamases and the marked synergic effect that it produces when it is administered associated with other β-lactamic antibiotics of the type of penicillins and cephalosporins.

It comes in the form of pharmaceutically acceptable salts and esters, which increase its stability and allow the same to be administered as such.

Among the salts which can be administered alkaline metal and alkaline earth metals and preferably sodium and potassium salts stand out. The suitable esters of clavulanic acid are the ones derived from alcohols and thiols, mainly those that can form the antibiotic of origin by gentle hydrolysis "in vivo."

This invention relates to a complete process of preparation of clavulanic acid from fermentation broths of Streptomyces sp. Said process has special characteristics, considered on the whole as well as in the isolated steps which encompass, and introduce numerous advantages over other methods of obtainment shown in the patents that are specified in the following section.

### PRIOR ART OF THE INVENTION

This invention refers to a process for the preparation of clavulanic acid and the pharmaceutically acceptable salts and esters thereof, which has noteworthy advantages over the methods described in Spanish patents 436,766,494,431 and over the improvements adduced in Spanish patent 550,133.

In effect, in the first one of them an extremely long and costly process is described and it requires laborious purifications based on different chromatographic methods which involve: initial filtrating of the culture broth which is difficult and which causes big losses; adsorption of the clavulanate anion over an anionic exchange resin; elution with an electrolyte; desalination of the resulting solution; application of the desalinated solution to an additional anionic exchange resin; chromatographic elution with an electrolyte; final desalination and, finally, elimination of the water or solvent used in the desalination. Aside from being very laborious, this process has the inconvenience that all the steps, with the exception of the last one which permits the use of organic solvents, are carried out in an aqueous solution in which clavulanic acid, and even its salts, are very unstable. The overall yield in raw sodium clavulanate represents approximately 20 % of that which is initially present in the culture filtrate.

Part of the purifications performed with resins are eliminated in Spanish patent 494.431 and the formation of a tert-butylamine salt is used as an intermediate step in the purification and the yield of this step is 75 %. However the prior steps of filtration of the culture broth or of isolation of the clavulanate anion over an anionic exchange resin are not eliminated and, consequently, neither elution with an electrolyte nor subsequent desalination are avoided. The crystallization of the tert-butylamine salt is done in an aqueous-acetonic solution and transformation thereof into sodium or potassium salt is obtained, either by passing an aqueous solution of the same through a cationic exchange resin or by treatment with the corresponding ethylhexaneate in an aqueous isopropanol solution. The overall yield is around 40 %.

Finally, Spanish patent 550.133 adduces improvements over the previous one which basically consist of the transformation of the tert-butylamine salt into alkaline salts, by treatment of the former with the sulfocyanide of the same metal as the one of the clavulanic acid salt that is to be obtained. The formation df the intermediate ter-butylamine salt is done by retroextraction of the organic solution of clavulanic acid with an aqueous solution of tert-butylamine at a pH of 5.5 with the subsequent danger of decomposition of the clavulanic acid given the high unstability of the same in aqueous solution (increased in this case by the acidity of the medium.) The use of alkaline sulfocyanides in the transformation of the intermediate tert-butylamine salt, though it is done in organic solvents, requires subsequent thorough elimination thereof.

### DESCRIPTION OF THE INVENTION

This invention relates to a process for the preparation of clavulanic acid and pharmaceutically acceptable salts and esters thereof from fermentation broths of Streptomyces sp. which comprises:

A) A step of prior "Flocculation" of the culture broth using inorganic electrolytes, preferably polyelectrolytes, as flocculation agents.

This method presents the following advantages:

- The "flocculation" is achieved in a spectacular way in a single step with the help of organic polyelectrolytes, generally synthetic polymers, more specifically the neutral ones and those of an anionic nature with negative residual charges.
- With this flocculation not only is the micelle separated, but also the proteins and particles in suspension initially present in the fermentation broth included in the flocculus are eliminated. The overall yields of this entire process are 85-90%.
- The flocculi that are formed in the culture broth are large and compact enough to allow their easy sedimentation and separation, which are more advantageously obtained using rolling sieves. Optionally the broth can then be subjected to a process of concentration applying a process of reverse osmosis.
- Thus a completely transparent culture broth is obtained without the need of using conventional methods such as adsorption over active carbon, the use of filtration coadjuvants and centrifugation. !n all of them it is subsequently necessary, unlike in this flocculation method, to subject the clarified culture broth to different processes of deproteinization and ionic exchange giving rise to big overall losses.
- Thus, in the case of Spanish patent no. 537,158 and European counterpart thereof EP-A-0 182 522, the use of filtration coadjuvants prior to adsorption over resins, does not eliminate the need of centrifugation or of deproteinization followed by further centrifugation. The deproteinization, upon being done at pH 4, necessarily entails a very considerable degradation of the clavulanic acid which, as has been reiterated above, is very unstable in an aqueous solution and this instability increases at an acidic pH. In effect, the overall losses accepted for this clarification process are 75-80%.
- In short, with the present "Flocculation" method the use of any type of ionic exchange resins, as well as the subsequent electrolytic displacements and desalinations are eliminated. This also avoids substantial losses aside from permitting the cost of the process to be lowered considerably and reducing remarkably the time needed for the purification of the clavulanic acid.

B) A steo of "Purification" of the clavulanic acid contained in the Streptomyces Sp. culture broth, flocculated and decanted in accordance with part A, which is characterized by the formation, as stable intermediate products, of a large variety of salts derived from primary, secondary and tertiary amines.

This process has the following advantages:

- It makes it possible to proceed with the direct extraction of the clavulanic acid from the flocculated culture broth, with different organic solvents by conventional methods without the need of subjecting it to any type of chromatographic separation.
- The formation and crystallization of the amine salts as intermediate compounds is performed from isolated organic solvents or a mixture thereof, without any amount of water intervening in any case and this prevents the degradation of the clavulanic acid, whose stability in the presence of water is much less.
- The intermediate compounds formed are clavulanic acid salts with a large variety of amines, having a high degree of purity (>85%) and their preparation is achieved with yields between 80-90%.

C) A step of "Transformation" of the intermediate salts formed from the clavulanic acid with the different amines according to the method described in B, by exchange with different alkaline and alkaline earth salts of a series of organic acids, including aromatic ones, whose pKa are higher than the pKa of clavulanic acid.

This method offers the following advantages:

- The use of ionic exchange resins in this step is eliminated, avoiding the contact of the salt with water and the subsequent losses.
- It is done exclusively in organic mediums without the presence of even minimal traces of water with which the most part of the degradation processes are eliminated.
- It permits preparation of the required pharmaceutically acceptable salts with yields around 90 % and a degree of purity of > 95 %.
- Pharmaceutically acceptable esters can be obtained from the clavulanic acid salts by direct estenfication of the salts following normal procedures.

The complete process for preparation of clavulanic acid has overall yields around 70 % from the Streptomyces clavuligerus fermentation broths The way in which the above cited steps (A) to (C) are carried out is specified hereinafter

A) Step of "Coagulation-Flocculation": This step allows separation of the proteins and particles in suspension initially present in the cultivated broth, retained in the flocculi. along with the micelle.

The size of the flocculi is large enough and they form in such a compact manner that their sedimentation and

separation turn out to be easy. The most advisable way to do so is to use rolling sieves

- Using this "Flocculation" step a completely transparent culture broth is obtained without the need of using conventional methods, such as adsorption over active carbon, the use of filtration coadjuvants and centrifugation, which subsequently entail deproteinization and ionic exchange processes, the cause of big overall losses.

  The "Flocculation" is carried out by means of the following sub-steps:

  A 1) Adding an inorganic electrolyte, such as alkaline and alkaline earth metal chlorides, or preferably ammonium chloride, to the culture broth, for the purpose of enhancing the action of the coagulant. Generally they are used in the proportion of 0.5-4 g/l of culture broth, however, the proportions may be varied in accordance with the consistency of the cultivated broth and the nature of the electrolyte.
  A 2) Incorporating, under stirring and keeping the pH between 6 and 8, preferably pH 7, an inorganic flocculant, generally hydrolyzable metal salts such as aluminum sulfate, ferric sulfate and preferably prehydrolyzed aluminum chlorides.
  When aluminum polychlorides are used, the $Al_2O_3$ content thereof should be between 10-20%. The necessary amount varies between 10-20 ml/l of fermentation broth.
  A 3) Adding, once the flocculation has started, an organic polyelectrolyte, or mixture of several, generally organic polymers, more specifically of the type of polyamines and preferably synthetic polyacrylamides of the suitable ionic nature. The pH necessary for the flocculating effect of these compounds to be the maximum is between 6 and 8 and the stirring as well as the duration thereof are determining factors in the formation and size of the flocculi. The aqueous solutions that are used of the different polyacrylamide countertypes are very diverse and the ones used the most are ones of concentrations between 0.01 and 0.2 % and preferably the ones of 0.05-0.1 % with a total concentration of 150-400 mg/l of fermentation broth.
  A 4) Separation of the flocculi from the fermentation broth, following rapid sedimentation, by means of using rolling sieves or by simple filtration, and once a completely transparent culture liquid has been attained, if the case so requires, prior concentration of said liquid by reverse osmosis may be proceeded with.

The comparative yields of the content of clavulanic acid in the flocculated culture broth with regard to the cultivated one are around 85-90 %.
B) Step of "purification" of the clavulanic acid contained in the culture broth flocculated and decanted in accordance with A which consists of the formation of a large variety of salts derived from primary, secondary and tertiary amines, giving rise to very stable intermediate products, which facilitates the direct purification of the flocculated broth without the need of subjecting it to any type of chromatographic separation.
This step consists of the following sub-steps:

B 1) Direct extraction with different organic solvents of the flocculated culture broth, cold and by means of adding an acid at a pH slightly lower than the pKa of the clavulanic acid, once the two phases are put and kept in contact with vigorous stirring. All conventional water immiscible solvents more advantageously the ones that have an average polarity, such as methyl, ethyl, propyl, isopropyl, n-butyl and isobutyl acetates, methylisopropyl ketone, methyl butyl ketone, methyl isobutyl ketone and n-butanol, are considered as suitable organic solvents.
B 2) Drying of the organic phase which contains the clavulanic acid on an inert desiccation agent such as $Na_2SO_4$, $MgSO_4$, etc.
B 3) Formation of the intermediate salt by adding the amine chosen among a large series of primary, secondary and tertiary amines, of general formula:

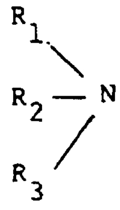

wherein $R_1$ can be H, $CH_3CH_3$-$CH_2$,

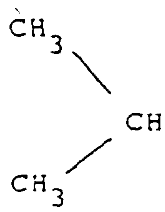,
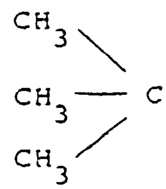

and in general $C_nH_{2n+1}$ (wherein n preferably has values between 1 and 7) and benzyl or substituted benzyl. or a cycloalkylic radical, wherein $R_2$ and $R_3$ can have, in isolation or simultaneously, the same values or can be linked together in such a way that the nitrogen forms part of a heterocycle.

The amine is added cold and dissolved in an organic solvent which may be of the same or different nature of the one used in the extraction of the active principle.

B 4) Stirring, keeping the mixture cold with which-a precipitate of the corresponding amine salt which, once filtered and dry, has a high purity (> 85 %) determined HPLC and a high stability, is formed. Thus it can be used as an intermediate in the purification process and as a starting point for the preparation of pharmaceutically acceptable salts and esters. The IR and $^1$H RMN spectra of the secbutylamine and benzylterbutylamine salts are described in examples 7 and 8 (Fig. 1, 2, 3 and 4) respectively.

The yield in the preparation of the intermediate amine salt, from the flocculated culture broth, is between 80 and 90 %, with which the overall yield in the purification is considerably improved with regard to that obtained by the methods stated in the above cited patents.

C) Step of Formation of pharmaceutically acceotable salts from the intermediate compounds obtained in accordance with B, by the interaction of the clavulanic acid contained in the culture broth flocculated with different amines, by exchange thereof with different alkaline and alkaline earth salts of a series of organic acids, R-COOH, including aromatic ones, whose pKa are higher than the pKa of clavulanic acid.

Acetic, trimetylacetic, propionic, isoproionic, butyric, isobutyric, phenylacetic acid, and in general all those branched alkyl and alkyl derivatives as well as the aromatic ones in which R is a phenyl or substituted phenyl group, such as is the case of benzoic, alkylbenzoic acids, etc., are considered as suitable starting organic acids. This step comprises the following sub-steps:

C 1) At the beginning the alkaline or alkaline earth salt, preferably the sodium or potassium salt of the chosen starting acid is formed by treatment of a solution of the acid in an organic solvent, chosen from the ones stated in part B, with the corresponding hydroxide, preferably sodium or potassium hydroxide, added to the solution in a stoichiometric amount. The mixture is stirred to homegeneity, the water released is eliminated by desiccation with an inert dehydrating agent, $Na_2SO_4$ or $MgSO_4$ preferably, and once this is filtered the volume is completed in order to obtain a standardized solution of the organic acid salt.

C 2) A determined amount of the intermediate amine compound is dissolved in the same or another organic solvent, chosen beforehand on the basis of the different solubilities of the starting salts and of the ones which are to be formed.

C 3) Once the two solutions, prepared according to C1 and C2, are cold, a determined volume of the one that contains the salt of the chosen acid is added over the one of the clavulanic acid salt with the amine, in such a way that the proportions of both are stoichiometric.

C 4) Upon mixing them cold a precipitate of the corresponding sodium or potassium salt of the clavulanic acid, whose crystallization is total after 1 h of stirring, begins to appear.

C 5) By filtration of the salt formed this way, sodium or potassium salts of clavulanic acid are obtained with a yield around 90 % in this transformation and with a purity higher than 97 %. The IR and $^1$H RMN spectra (Fig. 5 and 6) of the potassium salt obtained by following the described process are attached.

From the sodium or potassium salt pharmaceutically acceptable esters can be obtained by direct esterification thereof, following conventional processes, by reaction of the clavulanic acid salt with an esterifying agent, preferably halides, sulfonic esters, alcohols, etc., carried out in an organic solvent.

The various embodiments of the invention for which a protection is sought are featured in the appending claims.

DESCRIPTION OF THE FIGURES

Figure 1 : $^1$H RMN spectrum of the sec-butylamine clavulanate obtained in example 7.
Figure 2: The IR spectrum of the product of example 7.
Figure 3: $^1$H RMN spectrum of the benzylterbutylamine clavulanate obtained in example 8.
Figure 4: IR spectrum of the product of example 8.
Figure 5: $^1$H RMN spectrum of the potassium clavulanate obtained in example 27.

EMBODIMENTS OF THE INVENTION

Some illustrative examples of putting the present invention into practice, which cannot be considered as restrictive of its scope which is delimited exclusively by the set of claims, are described hereinafter.
The $^1$H RMN spectra have been carried out in a 300 MHz VARIAN VXR spectrometer, in solutions of approximately 2 % in $D_2O$. The chemical displacements (δ) are given in ppm and the coupling constants (J) are given in Hz.
The IR spectra have been carried out in a PERKIN ELMER® 881 apparatus separating the compounds in nujol.

EXAMPLE 1

An aqueous solution of an aluminum polyhydroxychloride (Prodefloc® AR-180), in a proportion of 1/70, following the addition of $NH_4Cl$ in the ratio of 1:1000 (w/v) is added to 250 ml of a Streptomyces clavuligerus fermentation broth which contains 1650 μg/ml of active principle and the flocculation which increases as the aqueous solution is neutralized with NaOH starts. Keeping the stirring vigorous, Praestol® 2540 at 0.05 % in a concentration of 250 mg/l, and water in the ratio of 1:5 (v:v), is added as an organic polyelectrolyte. Big flocculi appear and are separated from the supernatant liquid (with a concentration of active principle of 1405 mg/l with regard to the initial broth, from which Z(2R, 5R)-3-2-hydroxyethyliden)-7-oxo-4-oxa-1-azabicyclo-(3,2,0)-heptane-2-carboxylic acid can be directly extracted.

EXAMPLE 2

500 ml of a Streptomyces clavuligerus culture broth that has a concentration of 1025 mg/l of clavulanic acid is flocculated with the aluminum polychloride Prodefloc® AR-100 in the concentration of 30 ml/l. The $NH_4Cl$, which is used as an inorganic electrolyte is added in this case in the ratio of 3:2000 (w/v) The flocculi which are formed grow considerably after adding, following neutralization, Praestol® 2395 in an 0.1 % aqueous solution and in the ratio of 1:5 (v:v) After adding 50 ml/l of water, it is stirred vigorously for 2 minutes which produces a disintegration of the flocculi which facilitates their separation. The yield of active principle of the completely transparent yellow liquid represents 88 % of the initial one.

EXAMPLE 3

One liter of S. clavuligerus culture broth (1730 μg/ml of clavulanic acid) is flocculated with ammonium chloride and Prodefloc® AR-180 in a ratio of 1:100 (v:v) It is neutralized with NaOH and 170 mg of Praestol® 2500 in an 0.05 % aqueous solution. It is diluted with water and the flocculi are separated. The transparent liquid that is obtained is subjected to direct extraction of the clavulanic acid. The comparative concentration in active principle of the flocculated broth with regard to the fermentation broth is 1428 mg/l.

EXAMPLE 4

250 ml of Streptomyces clavuligerus fermentation broth with an active principle content of 2090 μg/ml is flocculated with $NH_4Cl$ (1 mg/l) and Prodefloc® AR 180 (14 ml/l), neutralizing with sodium hydroxide. The flocculi become larger upon adding 70 ml of 0.1 % Praestol® 2395 and 50 ml. of water. The separation of the flocculi permits to obtain a completely transparent liquid. The yield of active principle with regard to the cultivated broth is 86.4 %.

EXAMPLE 5

Two liters of fermentation broth which contain 950 μg/ml of Z(2R,5R)-3-(2-hydroxyethyliden)-7-oxo-4-oxa-1-azabicyclo-(3,2,0)-heptane-carboxylic acid are flocculated with 3.2 g of $NH_4Cl$ and 24 ml of Prodefloc® AR-180. NaOH

is added until neutralization and 400 ml. of an 0.1 % synthetic polyacrylamide solution, Magnafloc® 156, are added. The flocculi formed disintegrate with vigorous stirring facilitating the separation of the same which is favorably achieved by means of rolling sieves. The transparent liquid, which contains 2 mmol/l of active principle, can be concentrated by following a reverse osmosis process and then the same can be subjected to extraction with an organic solvent.

EXAMPLE 6

2 l of a S. clavuligerus culture broth which contains 1850 mg/l of clavulanic acid are flocculated with 2 g of ammonium chloride and 28 ml of Prodefloc® AR-180 as a coagulating agent. 560 mg of the flocculation coadjuvant, Praestol® 2395 in an 0.1 % aqueous solution are added to the previously neutralized suspension and then the same is neutralized with water and the stirring is maintained for a few minutes in order to facilitate the disintegration and separation of the flocculi. The yield is 90 %.

EXAMPLE 7

510 ml of the flocculate of example 4, which contains 1808 µg/ml of active principle referred to the fermentation broth are cooled at a temperature lower than 5°C and are put in contact, stirring vigorously, with a liter of ethyl acetate. Maintaining the stirring and the temperature HCl is added until the pH is approximately 2. The phases are separated and the aqueous solution is extracted again with one liter of ethyl acetate. The group of organic phases is dried upon $Na_2SO_4$ and the volume thereof is reduced to 14 ml. 2.37 mmol of cold secbutylamine in 14 ml of ethyl acetate are added and after one hour 500 mg of secbutylamine clavulanate with a purity of 85.2 % are separated. $^1$H RMN Spectrum (see Figure 1):

$\delta$

5'77 (1$H_{5\alpha}$ ) d ($J_{\alpha\text{-}\alpha}$= 2'8)
4'82 - 4'90 (2 $H_{(1'+2)}$) m
4'12 (2$H_{2'}$) d (J = 7'6)
3'60 (1 $H_{6\alpha}$) dd ($J_{9em}$= 17; $J_{\alpha\text{-}\alpha}$ = 2'8)
3'21 (1 $H_a$) q (J = 6'7)
3'06 (1 $H_{6\beta}$ ) d ($J_{gem}$= 17)
1'57 (2 $H_b$) m
1'21 (3 $H_{b,}$) d (J = 7'6)
0'93 (3 $H_c$) t ( J = 6'2)

IR spectrum (see Figure 2):

| | |
|---|---|
| 3260 $cm^{-1}$ | tens. O-H |
| 2600-2800 " | tens. $NH_3^+$ |
| 1790 " | tens. C=O β-lactamic |
| 1696-1684 " | tens. C=C exociclic |
| 1627 " | tens. COO- |
| 1574-1548 " | comb. tens. C-N +flex. N-H en $NH_3^+$ |

EXAMPLE 8

The flocculated fermentation broth coming from example 2 is extracted to an acid pH with 2 l of ethyl acetate keeping the mixture cold and with vigorous stirring. Once the organic phase is anhydrous and its volume has been reduced to 14 ml, 0.45 ml of cold benzylterbutylamine in 14 ml of ethyl acetate are added to it. Thus, benzylterbutylamine clavulanate is obtained with a yield of 85 % and a purity of 88.8 %. $^1$H RMN spectrum (see Figure 3):

$\delta$

7'42 (5 $H_c$) S
5'63 (1 $H_{5\alpha}$ ) dd ($J_{(5\alpha\text{-}6\alpha)}$=2'8; $J_{(5\alpha\text{-}6\beta)}$=0'7)
4'81 - 4'90 (2$H_{(1'+2)}$)m
4'14 (2$H_b$) S

(continued)

δ

4'11 (2 $H_{2'}$) d (J = 7'4)

3'47 (1 $H_{6\alpha}$) dd ($J_{gem}$ = 17; $J_{\alpha-\alpha}$= 2'8)

3'04 (1 $H_{6\beta}$) dd ($J_{gem}$= 17; $J_{(6\beta-5\alpha)}$= 0'7)

1'39 (9 $H_a$) S

IR spectrum (see Figure 4):

| | |
|---|---|
| 3334cm$^{-1}$ | tens. O-H |
| 2400-2800 " | tens. $NH_3^+$ |
| 1786 " | tens. C=O β-lactamic |
| 1687 " | tens. C=C exociclic |
| 1591 " | tens. C=C arom.+tens.COO- |
| 1580 " | comb.tens. C-N + flex. N-H en $NH_3^+$ |

EXAMPLE 9

510 ml of the liquid resulting from the flocculation of example 1 (687.5 mg/l) are cold extracted with 1 liter of ethyl acetate, keeping the mixture in contact by vigorous stirring and adding 0.38 ml of concentrated HCl. The aqueous phase is re-extracted with one liter of ethyl acetate and the volume of the organic solution is reduced to 10 ml. Then 0.20 ml of N,N-dimethylethylamine in 10 ml of acetone are added. After one hour of cold stirring 1.43 mmol of N,N-dimethylethylamine clavulanate crystallize with a purity of 87.5 %.

EXAMPLE 10

600 ml of the liquid coming from the concentration by reverse osmosis of the culture broth flocculated following example 5, which contain 2520.5 mg/l of clavulanic acid are extracted cold in the normal way with ethyl acetate (AcOEt) at an acid pH and once the organic phase is dried, the volume is reduced to 47 ml and 0.92 ml of cold 1.2-dimethylpropylamine in 47 ml of ethyl acetate (AcOet) are added. After stirring for an hour the corresponding clavulanic acid salt crystallizes. It has a purity of 84 % and it is obtained with a yield of 87 %.

EXAMPLE 11

4.12 liters of broth flocculated following the process of example 6 which contain 1628 µg/ml of clavulanic acid referred to the fermentation broth are concentrated to 500 ml following a process of reverse osmosis. It is extracted with ethyl acetate in the normal way and once the organic phase is dry it is concentrated to 94 ml and treated cold with 1.42 g of 1,1-dimethylpropylamine with which 14 mmol of the corresponding clavulanate, which has a purity of 85.5 %, crystallizes.

EXAMPLE 12

The flocculated culture broth of example 3 is subjected to a process of reverse osmosis reducing its volume to 540 ml with a content of active principle in the concentrate of 2485 mg/l. Careful cold extraction in ethyl acetate (AcOEt) is proceeded with keeping the two phases in contact, the temperature at 2 C and adding 0.75 of concentrated HCl. Once the ethyl acetate phase is dry, its volume is reduced to 40 ml and 0.80 ml of cold cyclohexylamine in 40 ml of ethyl acetate (AcOEt) are added. After 1 hour of stirring 1.55 g of the clavulanic acid salt with a purity of 84.6 % crystallizes.

EXAMPLE 13

500 ml of the liquid coming from the flocculation of the culture broth of example 2 are extracted in the normal way with ethyl acetate (AcOEt), and once the organic phase is dry it is reduced to 14 ml 0.27 g of cold neopentylamine previously dissolved in 14 ml of ethyl acetate (AcOEt) are added and after one hour 535 mg of neopentylamine clavulanate with a purity of 90 % are filtered.

EXAMPLE 14

510 ml of the broth flocculated in example 4 are extracted with ethyl acetate following the normal process. Once the volume of the organic phase has been reduced to 14 ml it is treated cold with 2.38 mmol of piperidine in 14 ml of ethyl acetate (AcOEt). After 1 hour the clavulanic acid salt formed which, once it is dry, weighs 0.57 g and has a purity of 82 %, is filtered.

EXAMPLE 15

1020 ml of culture broth flocculated following the method of example 2 are cold extracted at an acid pH with n-butanol. The volume is reduced to 14 ml and 390 mg of cold benzylterbutylamine in 14 ml of ethyl acetate (AcOEt) are added. Thus, 570 mg of benzylterbutylamine clavulanate with a purity of 83 % are obtained.

EXAMPLE 16

8.24 of the culture broth flocculated in example 6 are concentrated by reverse osmosis to 620 ml. They are extracted by cooling previously with methyl isobutyl ketone at an acid pH. The volume of the organic phase is reduced to 182 ml and 3.3 ml of cold secbutylamine in 182 ml of ethyl acetate (AcOEt) are added. Thus, secbutylamine clavulanate with a yield of 85.5 % and a purity of 90 % is obtained.

EXAMPLE 17

515 ml of the broth flocculated following the process of example 1 are cold extracted with 2 liters of methyl isobutyl ketone acidulating with 0.45 ml of concentrated HCl. The organic phase is dried and concentrated to 11 ml. 0.20 ml of N,N-dimethylethylamine in 11 ml of ethyl acetate (AcOEt) are added cold with stirring. 0.42 g of the corresponding clavulanate with a purity of 92 % are obtained.

EXAMPLE 18

500 ml of the culture broth of example 4 are flocculated following the cited process with which 1010 ml of a transparent liquid with a concentration of 895 mg/l of active principle are obtained. It is cold extracted with metyl isobutyl ketone following acidulation. Once the organic phase is dry, its volume is reduced to 27 ml, it is cooled and 4.8 mmol of benzylterbutylamine in 27 ml of ethyl acetate (AcOEt) are added crystallizing the clavulanic acid salt with benzyl terbutylamine. The yield is 92 % and the purity thereof is 95 %.

EXAMPLE 19

Two liters of S. clavuligerus fermentation broth are flocculated following example 2 and after concentrating by reverse osmosis to 550 ml an aqueous solution of clavulanic acid with a concentration of 3050 mg/l is obtained. It is extracted with methyl iso butyl ketone and once it is dry over $Na_2SO_4$ its volume is reduced to 50 ml. It is treated with 0.65 g of tert-butylamine in 50 ml of ethyl acetate and 7.58 mmol of the amine clavulanate with a purity of 90 % are obtained.

EXAMPLE 20

2010 ml of the broth flocculated in example 3 are extracted with methyl isobutyl ketone at an acid pH; once it is dry its volume is reduced to 43 ml. 7.5 mmol of cold 1,1 -dimethylpropylamine are added. After 1 hour of stirring 1.8 g of the corresponding clavulanic acid salt with a purity of 88 % crystallizes.

EXAMPLE 21

600 ml of the fermentation broth flocculated and concentrated by osmosis of example 5 are subjected to extraction with methyl isobutyl ketone in the normal conditions. It is dried, concentrated to 46 ml and 0.90 ml of cold cyclohexylamine in 46 ml of ethyl acetate (AcOEt) are added. 6.31 mmol of cyclohexylamine with a purity of 80 % are obtained.

EXAMPLE 22

From 515 ml of broth flocculated in example 4 extraction is done with methyl isobutyl ketone at a pH 2.1. The volume of the organic phase is reduced to 14 ml once it is dry and it is cold treated with 0.35 ml of 2-amino-3,3-dimethylbutane in 14 ml of ethyl acetate (AcOEt). 0.55 g of the amine salt of clavulanic acid with a purity of 90 % are obtained.

EXAMPLE 23

543 mg of the benzylterbutylamine of example 8 are dissolved in 75 ml of isobutanol and, once the solution is cool, 3 ml of an 0.5 M solution of potassium acetate in isobutanol are added while stirring and keeping the temperature below 5 °C. After stirring for 1 hour the potassium clavulante which is obtained with a yield of 91 % and a purity of 97 % crystallized.

EXAMPLE 24

An 0.2 M solution of potassium benzoate in butanol is prepared and 35 ml thereof are added over a solution of 2 g of 1,2-dimethylpropylamine clavulanate of example 10, in 20 ml of methanol (MeOH), kept cold and with stirring. Thus, 1.5 g of the potassium salt of clavulanic acid with a purity of 95 % crystallize.

EXAMPLE 25

408 mg of secbutylamine clavulanate of example 7 are dissolved in 30 ml of isopropanol. It is cooled to a temperature below 5 °C while stirring and 3 ml of an 0.5 M solution of potassium ethylhexanoate in isopropanol are added. After 1 hour of stirring in the cold, 350 mg of potassium clavulanate with a purity of 96 % crystallize.

EXAMPLE 26

An 0.05 M solution of N,N-dimethylethylamine clavulanate of example 9 in isopropanol is prepared. On 30 ml thereof and, cold, 15 ml of an 0.1 M solution of potassium trimethylacetate in isopropanol are added. The mixture is stirred and after 1 hour 375 mg of the potassium salt of clavulanic acid with a purity of 96 % are separated.

EXAMPLE 27

4 mmol of benzylterbutylamine clavulanate of example 18 are dissolved in 100 ml of isoamyl alcohol. The solution is cooled and 8 ml of an 0.5 M solution of potassium acetate in isoamyl alcohol are added over it. After 1 hour of cold stirring 900 mg of the potassium salt of clavulanic acid with a purity of 97 % are obtained. $^1$H RMN spectrum (see Figure 5):

$\delta$

5'69 (1$H_5$) d ($J_{5\alpha\text{-}6\alpha}$= 2'4)
4'88 - 4'96 (2H$_{(1'+2)}$)m
4'15 (2$H_{2'}$) d (J = 7'6)
3'52 (1 $H_{6\alpha}$) dd ( $J_{gem}$ =17; $J_{5\alpha\text{-}6\alpha}$ = 2'8)
3'09 (1 $H_{6\beta}$) d ($J_{gem}$ = 17)

IR spectrum (see Figure 6):

| | |
|---|---|
| 3326cm$^{-1}$ | tens. O-H |
| 1782 " | tens. C=O β lactamic |
| 1703 " | tens. C=C exociclic |
| 1604 " | tens. COO- |

EXAMPLE 28

6.8 g of the secbutylamine clavulanate of example 16 are dissolved in 600 ml of isopropanol. Once the solution is cool 50 ml of an 0.5 M solution of potassium acetate in isopropanol are added. After 1 hour of cold stirring potassium

clavulanate with a richness of 97 % and a yield of 94 % is obtained.

EXAMPLE 29

7.5 mmol of the terbutylamine clavulanate obtained in example 19 are dissolved in 350 ml of isobutanol and cold treated with 15 ml of an 0.5 M solution of potassium phenylacetate in isobutyl alcohol. After 1 hour of stirring, potassium clavulanate with a yield of 95 % and a purity of 96 % crystallizes.

EXAMPLE 30

14 ml of an 0.5 M solution of clavulanic acid salt and 1,1-dimethylpropylamine of example 20 are treated, cold stirring, with 14 ml of an 0.5 M solution of potassium acetate in ethanol. 1.5 g of potassium clavulanate with a purity of 97 % are obtained.

## Claims

1. A process for the preparation of clavulanic acid and pharmaceutically acceptable salts and esters thereof, from fermentation broths of Streptomyces sp., comprising flocculation of the culture broth, characterised in that the process comprises the following steps:

   A1) adding an inorganic electrolyte to the culture broth in the proportion 0.54 g/l.;
   A2) adding an inorganic flocculant, maintaining the pH between 6 and 8;
   A3) once the flocculation has started, adding an organic polyelectrolyte, in a concentration between 0.01 and 0.02%;
   A4) separating the flocculi from the fermentation broth and optionally concentrating the resultant broth by reverse osmosis;
   B1) directly extracting the broth obtained in process step A4 with an organic solvent at acid pH and treating the organic phase with an inert desiccating agent;
   B2) forming an intermediate salt of clavulanic acid with an amine of the general formula:

   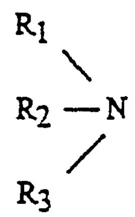

   wherein $R_1$ represents hydrogen, $C_nH_{(2n+1)}$ where n is an integer from 1 to 7, benzyl or substituted benzyl, or a cycloalkyl radical, and $R_2$ and $R_3$ independently represent the same values as $R_1$ or, together with the nitrogen atom form a heterocycle;
   C1) solubilising the amine salt intermediate formed in process step B2 in an organic solvent;
   C2) preparing a standard solution of an alkaline or alkaline earth salt of an organic acid having a pKa higher than that of clavulanic acid;
   C3) mixing the solutions prepared in process steps C1 and C2 in stoichiometric amounts at reduced temperature; and
   C4) optionally transforming the alkaline or alkaline earth salt of clavulanic acid formed in process step C3 to another pharmaceutically acceptable salt or ester.

2. A process as claimed in claim 1 wherein the inorganic electrolyte of process step A1 is an alkaline metal chloride. an alkaline earth metal chloride or ammonium chloride.

3. A process as claimed in claim 1 wherein the inorganic flocculant of process step A2 is a hydrolysable metal salt.

4. A process as claimed in claim 3 wherein the said metal salt is an aluminium polychloride having an $Al_2O_3$ content between 10 and 20%.

5. A process as claimed in claim 1 wherein the organic polyelectrolyte of process step A3 is a synthetic polyacrylamide.

6. A process for the preparation of clavulanic acid or a pharmaceutically acceptable salt or ester thereof, compnsing transforming an intermediate amine salt of clavulanic acid obtained by process step B2 of claim t into an alkaline or alkaline earth salt of clavulanic acid by reacting the amine salt with an alkaline or alkaline earth salt of an organic acid having a pKa higher than that of clavulanic acid and optionally transforming the alkaline or alkaline earth salt of clavulanic acid thus formed into another pharmaceutically acceptable salt or a pharmaceutically acceptable ester, subject to the proviso that the amine is not t-butylamine.

7. A process as claimed in claim 1 or 6 wherein the amine of general formula:

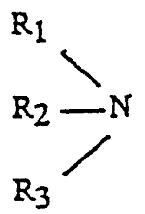

is sec-butylamine, benzylbutylamine, N,N-dimethylethylamine, 1,2-dimethylpropylamine, 1,1-dimethylpropylamine, neopentylamine, cyclohexylamine or piperidine.

8. A process as claimed in claim 1 or 6 wherein the process step of forming an intermediate amine salt is carried out under anhydrous conditions using organic solvents.

9. Use of an amine salt of clavulanic acid, obtained by process step B2 of claim 1, as an intermediate in the preparation of clavulanic acid and pharmaceutically acceptable salts and esters thereof, subject to the proviso that the amine is not t-butylamine.

10. Use of an amine salt of clavulanic acid as an intermediate as claimed in claim 9 when formed with 1,1-dimethylpropylarnine, sec-butylamine or cyclohexylamine.

**Patentansprüche**

1. Verfahren zur Herstellung von Clavulansäure sowie deren pharmazeutisch verträglichen Salze und Ester aus Fermentationsbrühen von Streptomyces sp., umfassend Ausflocken der Kulturbrühe, dadurch gekennzeichnet, daß das Verfahren die folgenden Schritte umfaßt:

   A1) Zugeben eines anorganischen Elektrolyts in einem Anteil von 0,5 - 4 g/l zur Kulturbrühe;

   A2) Zugeben eines anorganischen Mittels zum Ausflocken, das den pH-Wert zwischen 6 und 8 hält;

   A3) nach Beginn des Ausflockens, Zugeben eines organischen Polyelektrolyts in einer Konzentration zwischen 0,01 und 0,02 %;

   A4) Abtrennen der Flocken aus der Bermentationsbrühe und gegebenenfalls Anfkonzentrieren der entstandenen Brühe mittels Umkehrosmose;

   B1) direktes Extrahieren der in Verfahrensschritt A4 erhaltenen Brühe mit einem organischen Lösungsmittel bei saurem pH-Wert und Behandeln der organischen Phase mit einem inerten Trockenmittel;

   B2) Erzeugen eines intermediären Salzes der Clavulansäure mit einem Amin der allgemeinen Formel:

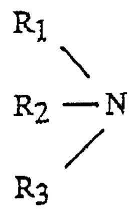

wobei $R_1$ ein Wasserstoffatom, $C_nH_{(2n+1)}$, in dem n eine ganze Zahl zwischen 1 und 7 ist, eine Benzylgruppe oder ein substituierter Benzylrest oder ein Cycloalkylrest bedeutet, und $R_2$ sowie $R_3$ unabhängig voneinander die gleiche Bedeutung wie $R_1$ haben oder zusammen mit dem Stickstoffatom einen Heterocyclus bilden;

C1) Löslichmachen des in Verfahrensschritt B2 erzeugten intermediären Aminsalzes in einem organischen Lösungsmittel;

C2) Herstellen einer Standardlösung eines Alkali- oder Erdalkalimetallsalzes einer organischen Säure mit einem höheren $pk_a$-Wert als Clavulansäure;

C3) Mischen der in den Verfahrensschritten C1 und C2 erzeugten Lösungen in stöchiometrischen Mengen bei reduzierter Temperatur; und

C4) gegebenenfalls Umwandeln des in Verfahrensschritt C3 erzeugten Alkali- oder Erdalkalimetallsalzes der Clavulansäure in ein anderes pharmazeutisch verträgliches Salz oder in einen Ester.

**2.** Verfahren nach Anspruch 1, wobei der anorganische Elektrolyt aus Verfahrensschritt A1 ein Alkalimetallchlorid, Brdalkalimetallchlorid oder Ammoniumchlorid ist.

**3.** Verfahren nach Anspruch 1, wobei das anorganische Mittel zum Ausflocken im Verfahrensschritt A2 ein hydrolysierbares Metallsalz ist.

**4.** Verfahren nach Anspruch 3, wobei das Metallsalz ein Aluminiumpolychlorid mit einem $Al_3O_3$-Gehalt zwischen 10 und 20 % ist.

**5.** Verfahren nach Anspruch 1, wobei der organische Polyelektrolyt aus Verfahrensschritt A3 ein synthetisches Polyacrylamid ist.

**6.** Verfahren zur Herstellung von Clavulansäure oder eines pharmazeutisch verträglichen Salzes oder Esters davon, umfassend Umwandeln des in Verfahrensschritt B2 gemäß Anspruch 1 erhaltenen intermediären Aminsalzes der Clavulansäure in ein Alkali- oder Erdalkalimetallsalz der Clavulansäure durch Umsetzen des Aminsalzes mit einem Alkali- oder Erdalkalimetallsalz einer organischen Säure mit einem höheren $pk_a$-Wert als Clavulansäure und gegebenenfalls Umwandeln des so erzeugten Alkali- oder Erdalkalimetallsalzes der Clavulansäure in ein anderes pharmazeutisch verträgliches Salz oder in einen pharmazeutisch verträglichen Ester, mit der Maßgabe, daß das Amin nicht t-Butylamin ist.

**7.** Verfahren nach Anspruch 1 oder 6, wobei das Amin der folgenden allgemeinen Formel

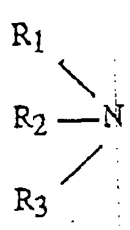

sec-Butylamin, Benzylbutylamin, N,N-Dimethylethylamin, 1,2-Dimethylpropylamin, 1,1-Dimethylpropylamin, Neopentylamin, Cyclohexylamin oder Piperidin ist.

**8.** Verfahren nach Anspruch 1 oder 6, wobei der Verfahrensschritt der Erzeugung eines intermediären Aminsalzes

unter wasserfreien Bedingungen mit organischen Lösungsmitteln durchgeführt wird.

**9.** Verwendung eines in Verfahrensschritt B2 gemäß Anspruch 1 erhaltenen Aminsalzes der Clavulansäure in der Herstellung von Clavulauäure und deren pharmazeutisch verträglichen Salze und Ester als Zwischenprodukt, mit der Maßgabe, daß das Amin nicht t-Butylamin ist.

**10.** Verwendung eines Aminsalzes der Clavulansäure nach Anspruch 9, das mit 1,1-Dimethylpropylamin, sec-Butylamin oder Cyclohexylamin erzeugt wird.

**Revendications**

**1.** Procédé pour la préparation d'acide clavulanique et de ses sels et esters pharmaceutiquement acceptables, à partir de bouillons de fermentation de Streptomyces sp., comprenant la floculation du bouillon de culture, ledit procédé étant caractérisé en ce qu'il comprend les étapes suivantes :

A1) addition d'un électrolyte inorganique au bouillon de culture en la proportion de 0,5 à 4 g/l ;
A2) addition d'un floculant inorganique, en maintenant le pH dans l'intervalle de 6 à 8 ;
A3) après le début de la floculation, l'addition d'un polyélectrolyte organique, à une concentration de 0,01 à 0,02 % ;
A4) séparation du floculat du bouillon de fermentation et, facultativement, concentration du bouillon résultant par osmose inverse ;
B1) extraction directe du bouillon obtenu dans l'étape de procédé A4 avec un solvant organique à pH acide et traitement de la phase organique avec un agent de dessiccation inerte ;
B2) formation d'un sel intermédiaire d'acide clavulanique avec une amine de formule générale :

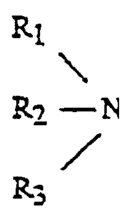

dans laquelle $R_1$ représente l'hydrogène, un radical $C_nH_{(2n+1)}$ dans lequel n est un nombre entier de 1 à 7, benzyle ou benzyle substitué, ou un radical cycloalkyle, et $R_2$ et $R_3$ ont, indépendamment, les mêmes valeurs que $R_1$ ou, en association avec l'atome d'azote, forment un hétérocycle ;
C1) solubilisation de l'intermédiaire consistant en le sel d'amine formé dans l'étape de procédé B2 dans un solvant organique ;
C2) préparation d'une solution de référence d'un sel de métal alcalin ou de métal alcalino-terreux d'un acide organique ayant un pKa supérieur à celui de l'acide clavulanique ;
C3) mélange des solutions préparées dans les étapes de procédé C1 et C2 en des quantités stoechiométriques à une température réduite ; et
C4) facultativement, transformation du sel de métal alcalin ou de métal alcalino-terreux d'acide clavulanique formé dans l'étape de procédé C3 en un autre sel ou ester pharmaceutiquement acceptable.

**2.** Procédé suivant la revendication 1, dans lequel l'électrolyte inorganique de l'étape de procédé A1 est un chlorure de métal alcalin, un chlorure de métal alcalino-terreux ou le chlorure d'ammonium.

**3.** Procédé suivant la revendication 1, dans lequel le floculant inorganique de l'étape de procédé A2 est un sel métallique hydrolysable.

**4.** Procédé suivant la revendication 3, dans lequel le sel métallique est un polychlorure d'aluminium ayant une teneur en $Al_2O_3$ de 10 à 20 %.

**5.** Procédé.suivant la revendication l, dans lequel le polyélectrolyte organique de l'étape de procédé A3 est un polyacrylamide synthétique.

**6.** Procédé pour la préparation d'acide clavulanique ou d'un de ses sels ou esters pharmaceutiquement acceptables, comprenant la transformation d'un sel d'amine intermédiaire d'acide clavulanique obtenu par l'étape de procédé B2 de la revendication 1 en un sel de métal alcalin ou de métal alcalino-terreux d'acide clavulanique par réaction du sel d'amine avec un sel de métal alcalin ou de métal alcalino-terreux d'un acide organique ayant un pKa supérieur à celui de l'acide clavulanique et, facultativement, la transformation du sel de métal alcalin ou de métal alcalino-terreux d'acide clavulanique ainsi formé en un autre sel pharmaceutiquement acceptable ou un ester pharmaceutiquement acceptable, sous réserve que l'amine ne consiste pas en tertio-butylamine.

**7.** Procédé suivant la revendication 1 ou 6, dans lequel l'amine de formule générale :

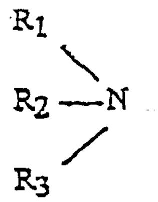

est la sec.-butylamine, la benzylbutylamine, la N,N-diméthyléthylamine, la 1,2-diméthylpropylamine, la 1,1-diméthylpropylamine, la néopentylamine, la cyclohexylamine ou la pipéridine.

**8.** Procédé suivant la revendication 1 ou 6, dans lequel l'étape de procédé de formation d'un sel d'amine intermédiaire est mise en oeuvre en milieu anhydre en utilisant des solvants organiques.

**9.** Utilisation d'un sel d'amine d'acide clavulanique, obtenu par l'étape de procédé B2 de la revendication 1, comme intermédiaire dans la préparation d'acide clavulanique et de ses sels et esters pharmaceutiquement acceptables, sous réserve que l'amine ne consiste pas en tertio-butylamine.

**10.** Utilisation d'un sel d'amine d'acide clavulanique comme intermédiaire suivant la revendication 9, lors de sa formation avec la 1,1-diméthylpropylamine, la sec.-butylamine ou la cyclohexylamine.

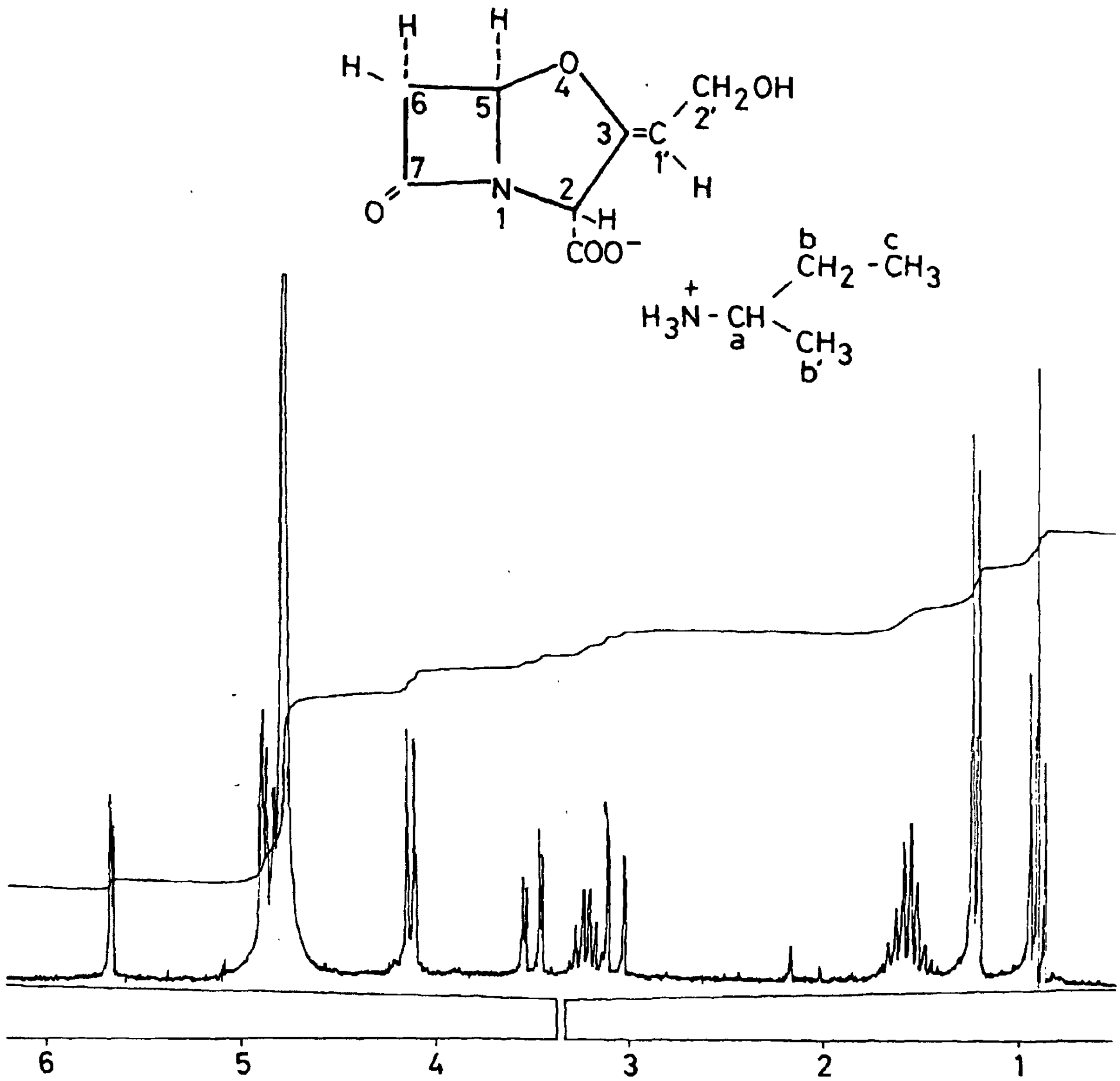


FIG-1

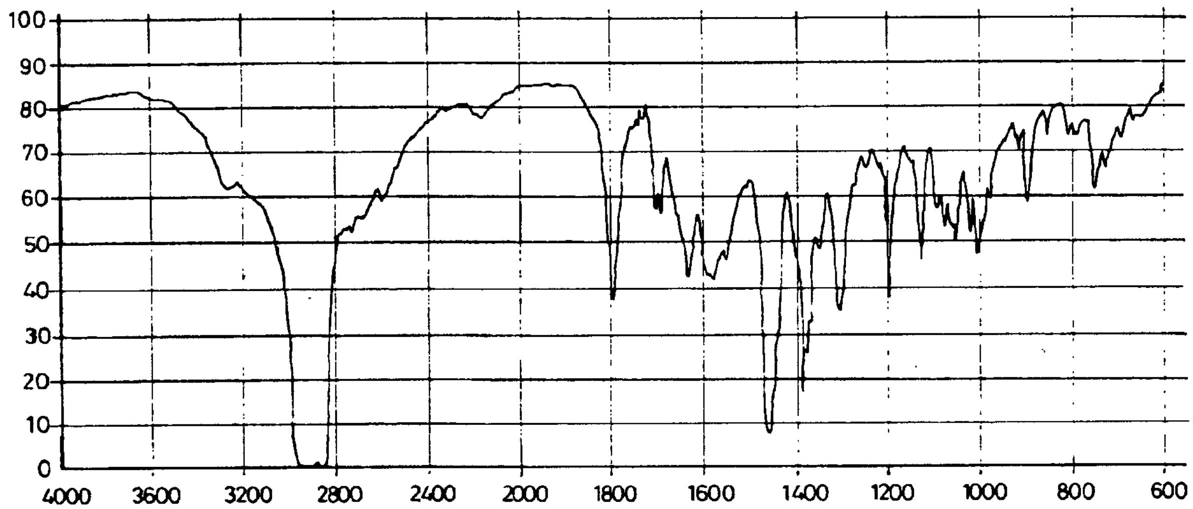
100
90
80
70
60
50
40
30
20
10
0
4000
3600
3200
2800
2400
2000
1800
1600
1400
1200
1000
800
600

FIG-2

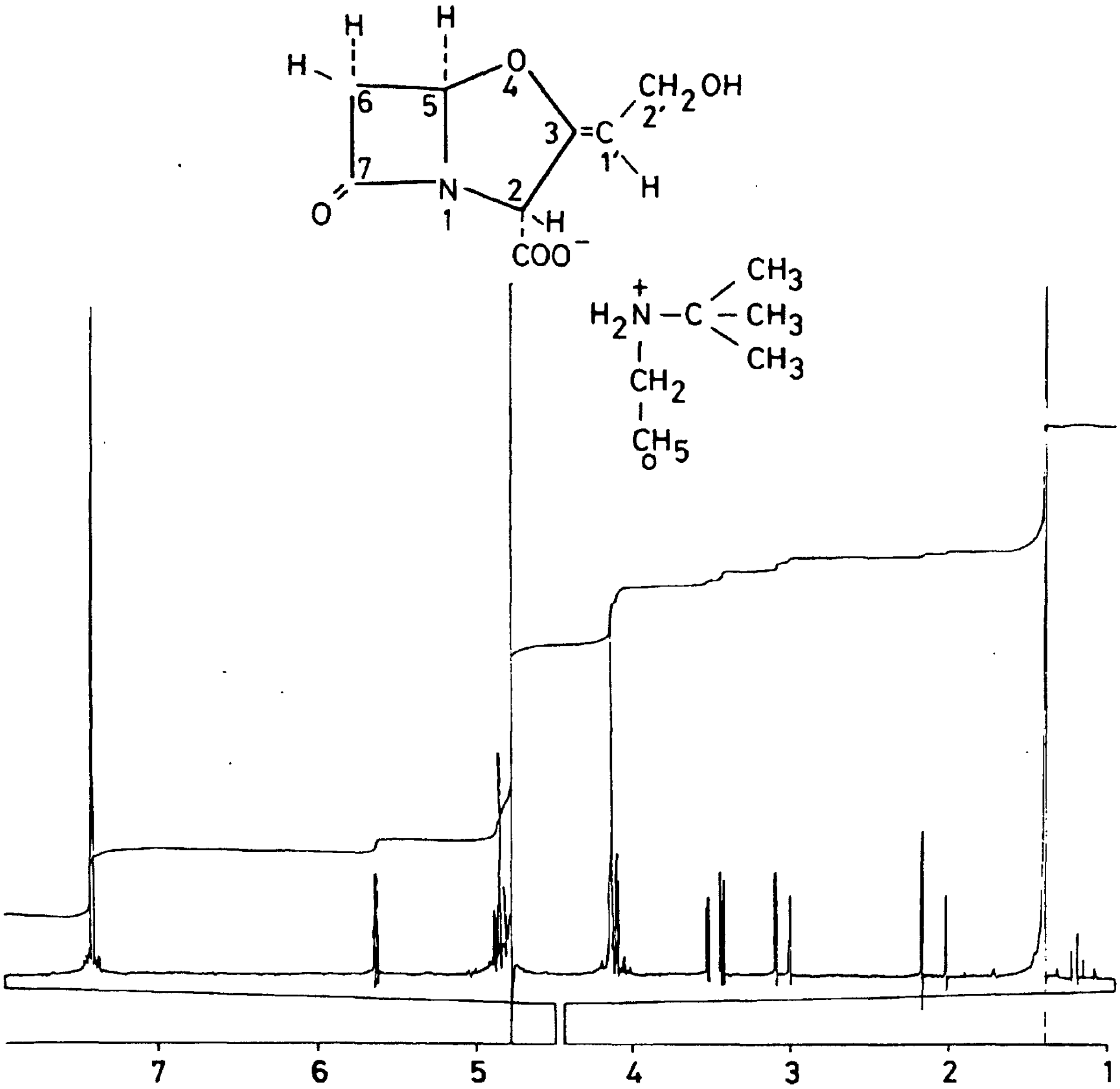


FIG-3

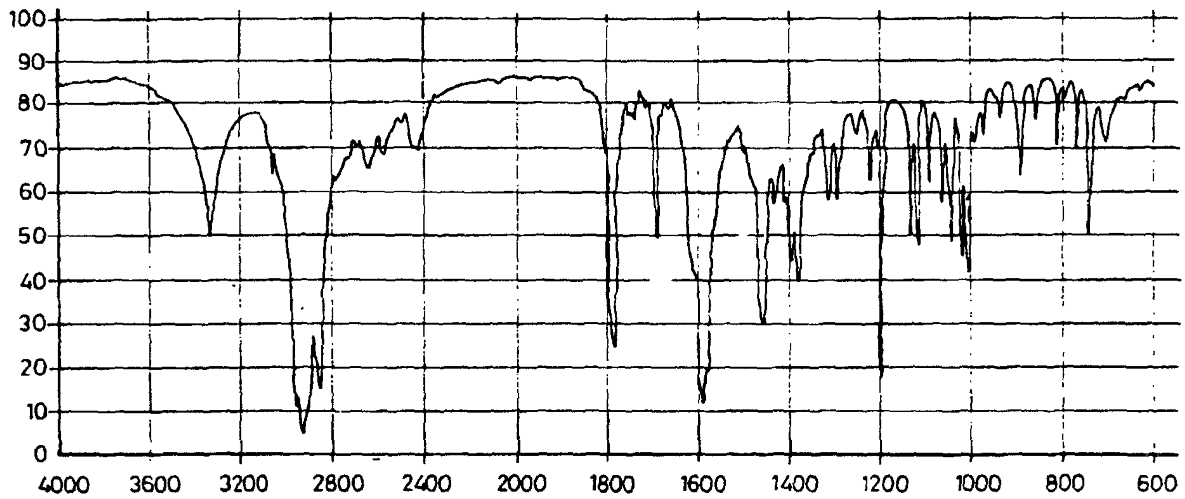
100
90
80
70
60
50
40
30
20
10
0
4000
3600
3200
2800
2400
2000
1800
1600
1400
1200
1000
800
600

FIG-4

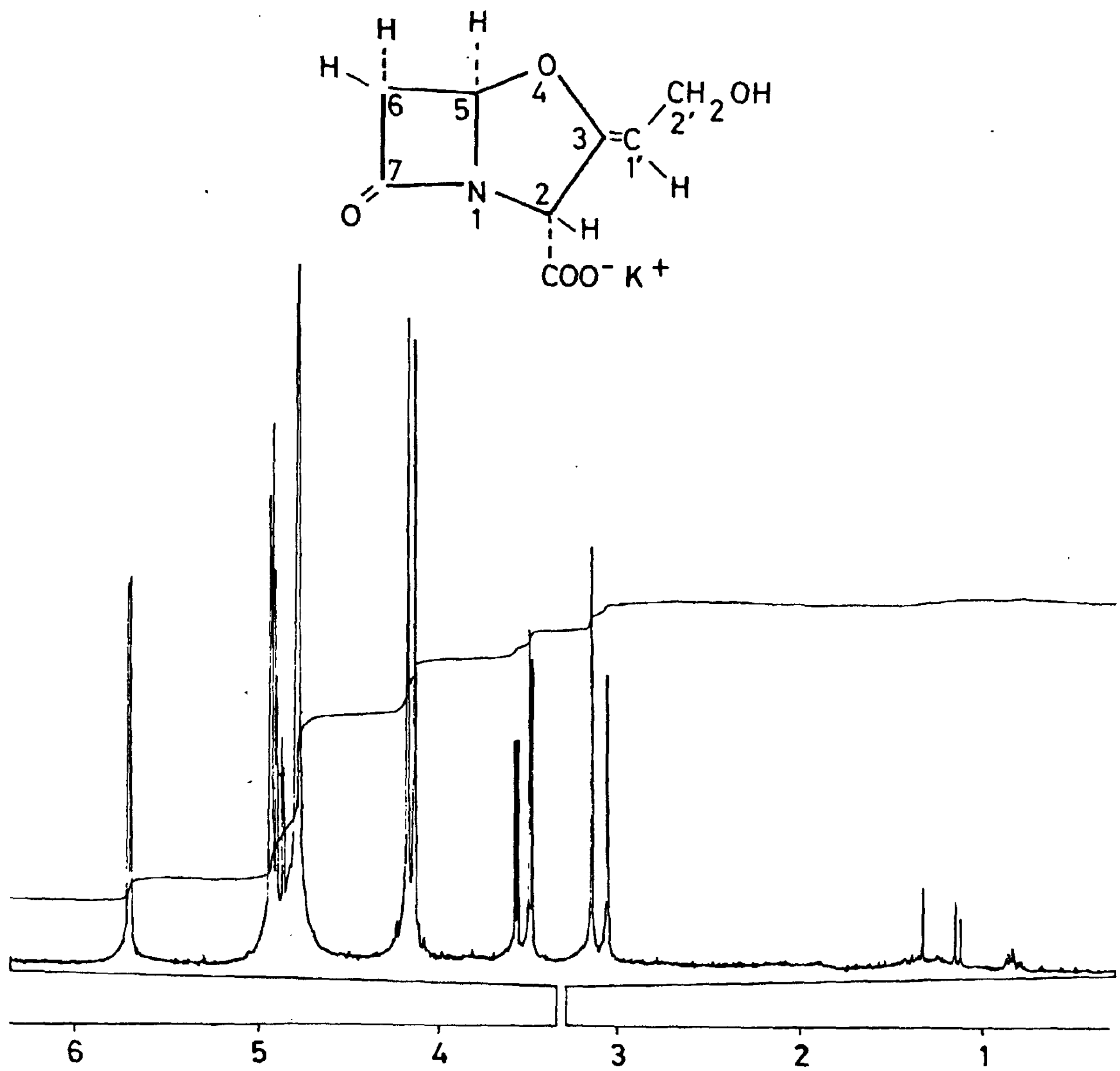

FIG-5

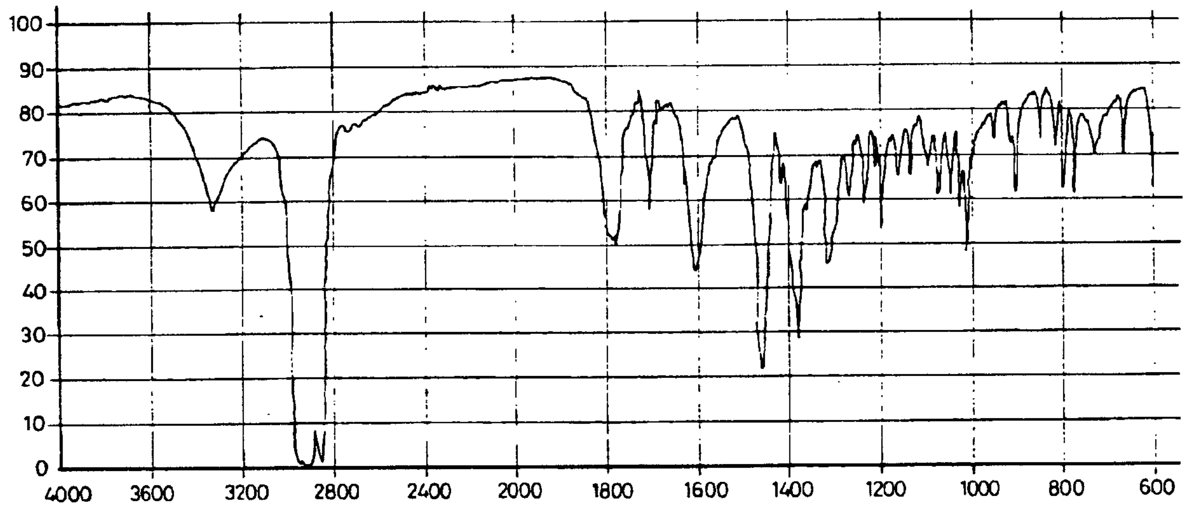
100
90
80
70
60
50
40
30
20
10
0
4000
3600
3200
2800
2400
2000
1800
1600
1400
1200
1000
800
600

FIG-6